# EUROPEAN PATENT APPLICATION

(11) **EP 4 715 735 A1**
(43) Date of publication of application: **25.03.2026**
(21) Application number: 25195851.8
(22) Date of filing: 14.08.2025
(51) Int. Cl.: G06T 7/00, G16H 50/70, G16H 30/40

(54) **COMPUTER-IMPLEMENTED METHOD OF COMPARING MEDICAL IMAGES OF A LONGITUDINAL STUDY**

(30) Priority: 19.09.2024 DE 102024127091
(71) Applicant: Siemens Healthineers AG, 91301 Forchheim (DE)
(72) Inventor: Abdishektaei, Mohammad, Charlottesville, 22903 (US); Chung, Ka-Man, 91052 Erlangen (DE); Hermosillo Valadez, Gerardo, West Chester, 19382 (US); Wolf, Matthias, Coatesville, 19320 (US); Yerebakan, Halid, Carmel, 46033 (US)
(74) Representative: Siemens Healthineers Patent Attorneys

(57) **Abstract**

The invention describes a computer-implemented method for generating a change map indicating at least one difference between a current image and a prior image. The method comprises
- obtaining a plurality of prior medical image sequences (30) from a prior study (30, 31), and a prior report (31) from the prior study (30,31);
- obtaining a plurality of current medical image sequences (20) from a current study;
- determining at least one medical finding based on the prior report;
- identifying, based on the determined medical finding, an image pair (14P, 14C), wherein the image pair (14P, 14C) comprises a prior image (14P) from one of the prior medical image sequences (30) and a current image (14C) from one of the current medical image sequences (20), and
- inputting the image pair (14P, 14C), and optionally the prior report (31), to a machine learning algorithm (16) trained to generate a change map (180) indicating at least one difference (180X) between the current image (14C) and the prior image (14P) of the image pair (14P, 14C).

## Description

The present invention relates to a method for generating a change map indicating a difference between images of an image pair.

Various medical imaging modalities can be used to identify an abnormality, for example a tumor or lesion. After identifying an abnormality in a medical imaging procedure, follow-up scans can be done at suitable intervals in order to assess its development. The images and reports of the initial scan and all follow-up scan(s) collectively comprise longitudinal data, and each set of medical images (and any reports) are referred to as a "study". A longitudinal study comprises multiple imaging sessions of the same patient over time.

Follow-up scans constitute approximately 75% of all medical imaging scans. For an imaging modality such as computed tomography (CT) or magnetic resonance imaging (MRI), a follow-up scan can comprise multiple series or sequences of images. Depending on the imaging protocols, a follow-up scan can comprise tens of images. The task of "reading" the images of a follow-up scan and comparing to one or more previous scans involves identifying the abnormality in the images of the current series, comparing with the abnormality as imaged in the prior scan(s), and assessing the consistency of the current observations with the prior report(s). This task can be very difficult in the case of only slight alterations of the depicted abnormalities. An inaccurate reading can have a detrimental effect on the patient's outlook, for example from a failure to identify a slight (but medically significant) deterioration in the follow-up scan. The task of reading the longitudinal data must therefore be carried out with great diligence in order to avoid misinterpreting the available information. It follows that comparing images of a longitudinal study is a time-intensive task which significantly adds to the workload of a clinician.

It is known to deploy machine-learning methods for the task of reading medical images. However, a problem with the known approaches is that the Al models used in such machine-learning tools are generally developed to perform a very specific task, e.g. to identify a lesion in an image. Such an Al model can only be used for that specific task, and is of only limited use to a clinician faced with the task of identifying developments in a longitudinal study.

It is therefore an object of the invention to provide an improved way of comparing longitudinal medical imaging studies. In particular, it is an object of the invention to enhance the visualization of changes of medical findings in longitudinal studies. In particular, it is an object of the invention to enhance the generation of change maps. In particular, the process of determining a change map shall be made more effective and reliable.

This task is solved by the features of the independent claims. Further advantageous examples are included in the dependent claims.

In the following specification, mostly, the explanations are limited to the field of MRI imaging. Of course, the explanations and the disclosed features are also applicable to other fields of imaging, in particular to other imaging modalities, e.g., CT imaging.

The invention is intended for use in comparing medical images of a longitudinal study, i.e. in assessing the medical images of a current study in view of a prior study, wherein the studies are preferably carried out for the same patient.

According to an aspect of the invention, a computer-implemented method for generating data indicating a change between a current image and a prior image, in particular for generating a change map indicating at least one difference between a current image and a prior image, is provided. The method comprises:
- obtaining a plurality of prior medical image sequences from a prior study, and a prior report from the prior study;
- obtaining a plurality of current medical image sequences from a current study;
- determining at least one medical finding based on the prior report;
- identifying, based on the determined medical finding, an image pair, wherein the image pair comprises a prior image from one of the prior medical image sequences of the prior study and a current image from one of the current medical image sequences of the current study, and
- inputting the image pair, and optionally the prior report, to a machine learning algorithm trained to generate data indicating a change between the current image and the prior image of the image pair, in particular a change map indicating at least one difference between the current image and the prior image of the image pair.

The medical images, i.e. the prior image and/or the current image, may be a two-dimensional image. The medical images may be a three-dimensional image. The medical images may be a four-dimensional image, where there are three spatial and one time-like dimensions. The medical images may comprise a plurality of individual medical images.

The medical image may comprise image data, for example, in the form of a two- or three-dimensional array of pixels or voxels. Such arrays of pixels or voxels may be representative of color, intensity, absorption or other parameters as a function of two or three-dimensional position, and may, for example, be obtained by suitable processing of measurement signals obtained by a medical imaging modality or image scanning facility.

The medical image may be a radiology image depicting a body part of a patient. Accordingly, it may contain two or three-dimensional image data of the patient's body part. The medical image may be representative of an image volume or a cross-section through the image volume. The patient's body part may be comprised in the image volume. Accordingly, the depicted body part of the patient in general will comprise a plurality of anatomies and/or organs. Taking a chest image as an example, the medical image may show lung tissue, the rib cage, lymph nodes and others.

A medical imaging modality corresponds to a system used to generate or produce medical images. For example, a medical imaging modality may be a computed tomography system (CT system), a magnetic resonance system (MR system), an angiography (or C-arm X-ray) system, a positron-emission tomography system (PET system) or the like. Specifically, computed tomography is a widely used imaging method and makes use of "hard" X-rays produced and detected by a spatially rotating instrument. The resulting attenuation data (also referred to as raw data) is processed by a computed analytic software producing detailed images of the internal structure of the patient's body parts. Magnetic Resonance Imaging (MRI), to provide another example, is an advanced medical imaging technique which makes use of the effect magnetic field impacts on movements of protons. In MRI machines, the detectors are antennas and the signals are analyzed by a computer creating detailed images of the internal structures in any section of the human body.

In the framework of this disclosure the term medical study shall be understand broadly, wherein a study comprises at least one medical image, in particular a set of medical images, and optionally, at least one medical report. A longitudinal study comprises multiple imaging sessions or studies over time. In particular, a longitudinal study may include a prior study and a current study. The images and reports of an initial scan and the follow-up scan(s) collectively comprise longitudinal data, which e.g., includes information about the course of a disease or the evolution of a medical finding.

Typically, a medical study includes multiple image sequences. In this context, a medical image sequence is to be understood broadly, wherein a medical image sequence relates to a particular setting of a medical imaging modality, which is used to acquire a medical image or multiple medical images. In particular, a sequence may be designed to highlight or enhance the visual appearance in a medical image of different aspects of tissue being examined. By using a variety of sequences, radiologists can obtain a comprehensive view of the area of interest, which helps in accurate diagnosis and assessment of medical conditions.

For example, an MRI study, e.g., a brain study, might include T1-weighted, T2-weighted, and/or FLAIR sequences, among others. Each of these sequences provides different types of information. As an example, T1-weighted sequences are particularly useful for providing a macroscopic appearance of tissues, in particular visualizing anatomical details. T2-weighted sequences are better for detecting fluid and edema, making them especially useful for identifying abnormalities like tumors or inflammation. Fluid attenuated inversion recovery (FLAIR) sequences are particularly good at highlighting lesions in the brain by suppressing the signal from cerebrospinal fluid.

Some imaging sequences may be contrast enhanced, in other words, the corresponding imaging sequences may be connected to the administration of contrast agent. For example, a CT or an MRI exam may include sequences for acquiring images before the administration of a contrast agent, also called pre-contrast sequences. Other medical image sequences may be used for acquiring medical images after the administration of contrast agent. In particular, a medical image or medical images may be acquired, when the level contrast agent at the location of interest is at its peak. A sequence for acquiring medical images in the period, when the contrast agent is washed out, may be called post-contrast sequence.

Some imaging sequences may include suppressing fat tissue. This means that the fat signal is suppressed in order to enhance the detectability of specific structural elements, such as lesions. Further, suppressing fat tissue may be used to prove that a certain tissue is fatty, as these tissue structures appear dark in a fat suppressed image sequence.

Preferably, the medical image is a 3D volume. In addition or as an alternative, the medical image may comprise a plurality of images or image slices. The slices may respectively show a cross-sectional view of the image volume. The slices may comprise a two-dimensional array of pixels or voxels as image data. The arrangement of slices in the medical image may be determined by the imaging modality or by any post-processing scheme used. Further, slices may artificially be defined in the imaging volume spanned by the medical image. Optionally, this may happen as a function of the image data comprised in the medical image in order to optimally pre-process the medical image for the ensuing diagnostic workflow.

The medical image may be a two-dimensional pathology image data set, i.e., a so-called whole-slide image. A whole-slide image may show a tissue slice or slide of a patient. The tissue slice may be prepared from tissue samples taken from the patient. Further, the preparation of a tissue slice may comprise the staining of the tissue slice with a histopathological staining. The staining in this case can serve to highlight different structures in the tissue slice, such as, e.g., cell walls or cell nuclei, or to test a medical indication, such as, e.g., a cell proliferation level. To create the whole-slide image, the stained tissue slices are digitized or scanned. To this end, the tissue slices are scanned with a suitable digitizing station, such as, for example, a whole-slide scanner, which preferably scans the entire tissue slice mounted on an object carrier and converts it into a pixel image.

The medical image may be stored in a standard image format such as the Digital Imaging and Communications in Medicine (DICOM) format and in a memory or computer storage system such as a Picture Archiving and Communication System (PACS), a Radiology Information System (RIS), and the like. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

A medical finding may relate to a corresponding medical image or a corresponding medical report. A medical finding may indicate a certain condition, abnormality and/or pathology of the patient. The condition, abnormality and/or pathology may be relevant for the diagnosis of the patient.

In other words, a medical finding may relate to an anatomical structure that differentiates the patient from other patients. Medical findings may be located within different organs of the patient (e.g., within the lung of a patient, or within the liver of a patient) or in between the organs of the patient. In particular, a medical finding may also relate to a foreign body.

In particular, a medical finding may relate to a neoplasm (also denoted as "tumor"), in particular, a benign neoplasm, an in-situ neoplasm, a malignant neoplasm and/or a neoplasm of uncertain/unknown behavior. In particular, a medical finding may relate to a nodule, in particular, a lung nodule. In particular, a medical finding may relate to a lesion, in particular, a lung lesion.

Medical findings may be classified according to their type or category. This type or category is called "finding type". A finding type may specify the general nature of medical finding. Further, the finding type may specify the anatomy or organ in which a medical finding has been found. According to some implementations, the finding type may also be conceived as a label of the medical finding. For instance, finding types may be lung nodule, liver nodule, cyst, rib fracture, undefined lesion, etc. In some cases, the medical finding may be that there is no finding in the medical image data.

A medical finding may be determined by a human evaluator of the medical image data and/or by a computer aided detection algorithm, which may generally be configured to detect candidate medical findings in medical images. For instance, the findings detection algorithms may have two stages: the detection stage for detecting potentially relevant patterns in image data and the classification stage for classifying the potentially relevant patterns either as candidate medical findings or as false positives to be discarded. In principle, a plethora of functionalities and methods is known for such computer aided detection and classification of candidate medical findings - all of which may be implemented in the findings detection algorithms.

In general, "Obtaining" data may include "collecting" data and/or "determining" data and/or "receiving" data and/or "retrieving" data and/or "querying a database to obtain" data, independently whether the data is embodied as query data or as knowledge data. Furthermore, "Querying a database to obtain data" may include the step of "obtaining data".

The at least one medical finding may be included in a medical report. The term medical report shall be understood broadly, and refers to a documentation of medical data, in particular consultations, medical examinations, test results, medications taken, and/or progress reports. The medical report may provide information regarding a patient's medical history, current health condition, clinical findings, treatments administered, and/or the progress or prognosis of a person's health status. In particular, a medical report may include, e.g. textual passages, images, audio transcription and/or 4D, i.e. video data. Preferably, the medical report may be an HL7 report.

At least one medical finding may be included in the medical report in textual form, as an image, e.g. an image with segmentation, and/or in any other form. The medical report may be processed to extract the at least one medical finding. For this, in particular, when the medical report includes a textual medical finding, the textual report may be inputted to a language model, in order to extract at least one medical finding from the medical report. In particular the language model may be capable of performing text mining on the prior report to obtain the at least one medical finding.

The language model may comprise a deep neural network employing, for example, a transformer architecture. The language model may be a large language model having a large number of neurons, such as one billion neurons or more, preferably 7 billion neurons or more, more preferably 13 billion of neurons or more, and most preferably 70 billion neurons or more. In particular, the language model may be configured to receive a sequence of tokens, such as words, syllables, or lexemes, as input and to output one token as output that is deemed to be the "most probable" next token. In particular, the language model may be operated in a generative or autoregressive manner, that is: an entry being a sequence of tokens may be provided to the language model as input, the first most probable next token output by the language model may be appended to the entry to obtain a new entry to be provided to the language model as input in the next iteration, and by repeating this sequence of iterations, the language model may generate a meaningful most probable response to the entry that comprises several sentences or even paragraphs.

Thereby, the language model may be trained and/or prompted to extract at least one medical finding from the medical report, in particular to generate a list of findings of the medical report. The language model may also be able to determine contextual information for medical findings.

Preferably, the language model may be a foundation model capable of processing multi-modal input data, such that the model can process textual inputs as well as input image data.

In particular, the language model can be pre-trained so as to understand natural language and have the capability to give meaningful responses about an arbitrary subject. Such pre-training is possible by using a text database that is the result from crawling a portion, preferably a substantial portion, of the Internet as language training data. As such, no labelling of the language training data used for the pre-training is required. However, the pre-training of the large language model may, optionally, also comprise steps of supervised training using labelled language training data generated by humans so as to further improve the capability of the large language model to provide meaningful responses to the user.

Examples for a pre-trained generative language model include OpenAl's ChatGPT 3 and 4, Google Al's PaLM, BERT, and Gemini, DeepMind's Chinchilla, and Meta's Llama 1, 2 and 3 models. The language model may be trained from scratch or by fine-tuning an existing model, e.g., using supervised learning, unsupervised learning, or semi-supervised learning techniques. In particular, the language model can be fine-tuned for the tasks to which it is going to be applied according to the proposed solution. That is, fine-tuning may comprise unsupervised or supervised further training of the language model, using language training data that relates to the suggested system.

For example, the language model may be trained using a labeled training dataset. The labeled training input data may include medical reports including medical findings.

The language model may be trained using backpropagation techniques. For example, different entries (e.g., prompts, training samples of medical reports) in a training dataset may be provided to the language model and a list of the included findings may be generated.

A difference between the output of the language model and a label for the entry may be determined according to a loss function. Based on the difference, backpropagation techniques may be used to adjust the parameters and/or weights of the language model. The language model may be trained in this manner over time with different labeled entries, e.g., until the language model is accurate to an accuracy threshold. At this point, the language model may be deployed for use to extract medical findings from medical reports.

The identifying of the image pair may be based on specific characteristics of the image sequences, to which the prior image and the current image respectively correspond. In particular, it may be determined, which image sequences are suitable for visualizing the determined medical finding. In particular, it may be determined, which image sequences are most suitable for being compared. In particular, the prior image and/or the current image may be selected such that the characteristics of the corresponding prior image sequence and/or the corresponding current images sequence are suitable for identifying the at least one determined medical finding. E.g., images from a post-contrast sequence may be used as the image pair, if the determined medical findings include a tumor. Also, a combination of the aforementioned approaches may be applied.

Characteristics of an image sequences may include, e.g., the image modality used, the architecture of the sequences, e.g., excitation pulses and gradients for spatial encoding, administration of contrast agent, reduction of artifacts, filtering options, including fat saturation, used echo types, chosen sequence parameters like flip angle, field of view matric, etc. Other characteristics which may be used are, for example, the resolution, i.e. the level of detail that the imaging sequence can capture, wherein the resolution may be spatial and/or temporal, the field of view (FOV), i.e. the extent of the area being imaged, which can vary depending on the clinical requirement, e.g., whole-body imaging vs. localized imaging, the slice thickness, the Signal-to-Noise ratio (SNR), i.e., the level of signal relative to the background noise, which affects the clarity and quality of the images, the patient positioning, i.e., the orientation and position of the patient during the imaging process, which can influence the quality and diagnostic value of the images, and/or the applied post-processing techniques, such as methods used to enhance or analyze the images after acquisition, such as 3D reconstruction, filtering, or segmentation.

In other words, the method may include identifying the prior and/or current medical sequences which are most appropriate for visualizing the determined medical finding. In particular, whether a medical sequence is determined as appropriate for visualizing a medical finding may depend on whether the determined medical finding is identifiable in the corresponding medical image, or how well the determined medical finding is identifiable in the corresponding medical image. For example, there are specific sequences in which soft tissue is better detectable than bones. Other sequences may be better for detecting bones than for detecting soft tissue. Some sequences may include contrast agent and therefore may be particularly suitable for detecting certain medical findings. In addition or as an alternative, the method may include identifying the prior and/or current medical sequences which are most suitable for being compared.

Within the framework of this disclosure, the term change map shall be interpreted broadly and relates to a medical image, visually indicating the optical change between a prior image and a current image. There are various algorithms which are known for detecting and visualizing changes between images.

The data indicating a change between the images, in particular the change map, may be generated by a machine learning algorithm. In general, a machine learning algorithm mimics cognitive functions that humans associate with other human minds. In particular, by training based on training data, the machine learning algorithm is able to adapt to new circum-stances and to detect and extrapolate patterns. Other terms for machine learning algorithm may be machine-learned function, trained function, trained machine learning model, trained mapping specification, mapping specification with trained parameters, function with trained parameters, algorithm based on artificial intelligence, or machine learned algorithm.

In general, parameters of a machine learning algorithm can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning algorithm can be adapted iteratively by several steps of training.

In particular, a machine learning algorithm can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

For instance, the machine learning algorithm may be configured to extract one or more features from the input data, in particular from the medical images, and map/classify these features into a feature space associated with different finding for determining, which finding the extracted features indicate.

The usage of machine learning algorithms in general has the advantage that a more comprehensive and faster screening of the available information can be made. In this regard, machine learning algorithms may identify patterns and attributes in the available data that are not accessible for a human.

As the image pair is selected based on the determined medical finding, it is ensured that the medical finding is well detectable in the images forming the image pair. Thus, the detectability of the medical finding in the change map is improved and the workflow for medical evaluations of the images is enhanced.

As findings can be automatically extracted from the medical report, the inventive approach allows for a pre-loading of the most suitable and/or most relevant change maps, such that a user can quickly switch between multiple change maps without delay. The images for comparison are selected based on the medical finding, in particular based on the characteristics of the respective image sequences, such that the medical findings are detectable and such that background noise is reduced. Thus, the time a radiologist requires for generation and investigation of the change map(s) is shortened, as there is no need for the radiologist to first determine a suitable image pair based on which a change map can be calculated.

According to an embodiment, the method comprises a step of outputting the change map to a user and/or providing the change map for further processing. Due to the improved visualization of changes in medical findings, the change map may be directly interpretable by a radiologist and may therefore be directly forwarded to a user. Further processing may include applying image filters to the generated change map, e.g. if multiple deviating visualizations shall be generated based on the change map. In particular, the change map may be displayed.

The data representing the change, in particular the change map, may be used by a medical professional in a variety of ways. For example, the medical professional may recommend a course of treatment, or the professional may decide that further follow-up imaging is required. According to an example, a visualization of the change map may be displayed. Due to the enhanced detectability of the medical findings in the medical image pair based on which the change map is calculated, the change map objectively provides a better visualization and therewith understanding of the medical findings.

According to an embodiment, the method comprises a step of performing text mining on the prior report to obtain or determine the at least one medical finding. Preferably, the text mining may include inputting the prior report or parts of the prior report to a language processing algorithm, in particular a language model, to determine the medical finding included in the report. Preferably, if the report includes a plurality of medical findings, a list of medical findings may be generated. Thus, medical findings are automatically extracted from the prior medical report. This makes the method more efficient and reliable.

According to an embodiment, the prior image is determined based on the medical finding and the current image corresponds sequentially to the prior image. Vice versa, it is also possible to select the current image based on the determined medical finding and to further select the prior image which is most suitable for comparison with the current image. Thus, the step of selecting an appropriate image sequence and based on that, the corresponding medical image may only be conducted for the prior medical image or for the current image. The respective other medical image may be already assigned to the selected medical image. In other words, a plurality of medical image pairs may be available, wherein each image pair includes a prior medical image and a current medical image. The image pairs may be build based on the corresponding image sequences, such that the images constituting an image pair may match each other as closely as possible. Then, based on the determined medical finding, an appropriate medical sequence may be identified, and the image pair corresponding to the image sequence may be selected.

According to an embodiment, the method comprises a step of generating a context for a difference indicated in the change map based on the image pair, based on the prior report and/or based on the determined at least one medical finding. Preferably by means of the machine learning algorithm and/or the language model, wherein, preferably, the machine learning algorithm and or the langue model is trained to generate the context for at least one of the determined findings. This context may then be used for explaining a difference indicated in the change map. The generated context may be output in textual form, stand-alone or in combination with the change map, to enhance the understanding of change map. Based on the context, a report may be compiled, describing the longitudinal evolution of the determined medical finding. The complied report may be a structured report.

In an example, the generated context may include at least one of the following information:
- where is a change and/or finding located in the image data;
- where is a finding described in the prior report;
- what has changed between the prior study and the current study;
- to which extend have the findings changed;
- patient information available in the prior report, such as age, size, weight, etc
- other related findings of the patient, such as findings in other organs
- etc.

Thus, according to an embodiment, the method comprises a step of compiling a report for the current study based on the change map, and optionally based on the provided context. The report may include visual elements, in particular the prior image, the current image and/or the change map, and/or textual elements, such as the determined medical finding, the generated context. The generated report may be output for further processing or for signing off by a radiologist. Thus, the workflow when investigating a change map or change of findings in longitudinal studies is enhanced.

As explained earlier, preferably, the prior image and/or the current image is a 3D medical image. Further preferably, the 3D images are input into the machine learning algorithm for generating the change map, and a 3D change map is generated based on comparison of the 3D data. Based on the 3D change map, a plurality of slices, i.e. 2D images, may be computed, such that a viewer of the 3D change map can quickly switch between slices without the need of repeating the calculations of the 3D change map.

According to an embodiment, the method comprises a step of obtaining a raw image set of the prior study and/or a raw image set of the current study. The method may further comprise a step of sorting the medical images of the raw image set into medical image sequences, based on the sequence used to acquire the medical images. In particular, the sorting may be based on the metadata of the medical images included in the raw image set. Medical imaging sequences are highly specific for the respective vendor or user of imaging modalities. For example, similar imaging sequences may have different names or imaging sequences with similar names may include different settings. Therefore, determining which imaging sequences are available in the prior study and the current study, and/or determining how the imaging sequences correspond to each other, is crucial for providing a base for a reliable change map generation process.

Further, as already shortly explained above, medical image pairs may be determined, wherein each pair includes a medical image from a prior study and an image from the current study. According to an example, the pairs may be determined based on the medical sequences used to acquire the medical images. Thus, for a prior medical image it may be determined, which current medical image was acquired with a similar image sequence. These two medical images may form an image pair. The similarity of the medical images and/or the image sequences may be determined based on the characteristics of the image sequences. Thus, multiple medical image pairs may be prepared, and a quick selection of the medical image pairs based on the determined medical finding may be conducted.

According to an embodiment, the method comprises a step of conducting image registration on the prior image and/or on the current image. With other words, an image registration between the prior image the current image may be conducted. Image registration is the process of transforming different sets of medical imaging data into a common coordinate system to facilitate the comparison of the medical images and the generation of the change map. Conducting image registration before inputting the medical images into the machine learning algorithm facilitates the generation of the change map and therefore improves the results, in particular the quality of the generated change map is enhanced.

According to an embodiment, the prior medical image and/or the current medical is obtained by magnetic resonance imaging. Alternatively or in addition, the prior medical image and/or the current medical image may be obtained by CT imaging, X-ray imaging or any other image technology.

According to an embodiment, the prior study and/or the current study was conducted under administration of a contrast agent, and the prior image and/or the current image is further identified based on the contrast setting of the corresponding image sequence. As stated before, the administration of contrast agent is one of the characteristics of a medical image sequence and therefore is suitable for determining the suitability of the current image and/or the prior image to visualize the determined medical finding.

The prior report may include a plurality of medical findings. According to one embodiment, a summary of medical findings is generated based on the prior report. The summary may include all of the medical findings or some of the medical findings included in the prior report. Then, the method may include looping/iterating over the findings in the summary of medical findings. At least one of the following steps is repeated for each of the medical findings in the summary of medical findings:
- identifying, based on the determined medical finding, an image pair, wherein the image pair comprises a prior image from one of the medical image sequences of the prior study and a current image from one of the medical image sequences of the current study;
- inputting the image pair, and optionally the prior report, to a machine learning algorithm trained to generate a change map indicating at least one difference between the current image and the prior image of the image pair; and
- outputting the change map to a user or providing the change map for further processing.

In other words, for each of the medical findings in the summary, the most suitable image sequence may be determined. Based on that, the most appropriate image pair may be identified. Thus, there is one image pair for each medical finding. A change map may be generated based on the respective image pair. As a result, a radiologist may be able to view the most appropriate change map for each of the findings.

In particular, the respective change maps may be prepared before a user starts evaluating the first change map. Thus, a quick switching between the already prepared change maps can be accomplished avoiding delays in the diagnostic workflow due to loading or calculation of new change maps.

According to a further aspect, a data processing apparatus comprising means for carrying out the steps of the method according to any of the claimed embodiments is provided.

According to a further aspect, a computer program product is provided, wherein the computer program product comprises instructions, which, when the program is executed by a computer, cause the computer to carry out any of the above-mentioned embodiments.

The computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a further aspect, a computer-readable storage medium is provided, comprising instructions, which, when executed by a computer, cause the computer to carry out the steps of the method according to any of the above-mentioned embodiments.

According to an embodiment, the computer-implemented method comprises steps of: identifying an image pair, wherein an image pair comprises a first image from a medical image sequence of a prior study and a second image from a medical image sequence of a current study, and wherein the first image is selected on the basis of an indication noted in the prior study, and the second image corresponds sequentially to the first image (e.g. both images show the same "slice" through the patient's body; both images are volumetric images showing the same body regions of the patient). The method comprises a subsequent step of inputting the image pair to a machine learning algorithm previously trained to generate a change map indicating any difference between the current image and the prior image of the image pair; and outputting the change map to a user.

According to an embodiment, the method of assessing the medical image sequence of a current study comprises the steps of obtaining a prior study with an older medical image sequence and a report of noted indications; processing the image sequences using the claimed computer-implemented method; and compiling a report for the current study on the basis of the change map. The change map can also assist the radiologist in reading the image(s) from the current study.

According to an embodiment, the data processing apparatus is adapted to perform the steps of the claimed computer-implemented method and comprises at least an input interface for receiving a medical image sequence of a current study; a means of obtaining the prior study; a means of generating an indication summary for the prior study; and an image pair selector configured to compile an image pair as defined above. The data processing apparatus further comprises an instance of a machine learning algorithm configured to receive an image pair and trained to generate a change map indicating any difference between the current image and the prior image of an image pair. The data processing apparatus further comprises a means of outputting the change map to a user.

According to an embodiment, the method of training the machine learning algorithm of the claimed data processing apparatus comprises at least the steps of generating an image pair by obtaining a medical image; and manipulating the intensity of the image to obtain an intensity-manipulated version of the image; inputting the image pair to a machine-learning algorithm;
applying self-supervised learning to train the machine-learning algorithm to identify differences between the image pair.

An advantage of the inventive approach is that, by subjecting an image of an image pair to the intensity transformation, any intensity variations arising from hardware differences are essentially factored out and can no longer have a negative influence on the subsequent stages of image comparison. The inventors have realized that problems with the known approaches can arise from unavoidable intensity variations between the current and prior image sequences, for example when the MRI hardware used to generate the prior image set is not the same as (or was configured differently than) the MRI hardware used to generate the current image set. By factoring out such intensity variations, the inventive method presents a favorably robust way of identifying relevant changes between medical image sets.

The object of the invention is also achieved by a computer program product with a computer program that is directly loadable into the memory of a data-processing apparatus, and which comprises program units to perform the steps of the inventive computer-implemented method when the program is executed by the control unit.

Particularly advantageous embodiments and features of the invention are given by the dependent claims, as revealed in the following description. Features of different claim categories may be combined as appropriate to give further embodiments not described herein.

The imaging modality used to obtain the medical image sequence can be computed tomography (CT), magnetic resonance imaging (MRI), positron emission tomography (PET), single-photon emission computed tomography (SPECT), X-ray imaging, ultrasound imaging, photoacoustic imaging, etc.

A report compiled by a clinician to accompany a medical image sequence generally includes various types of information, among which might be one or more indications or findings identified by the clinician after viewing the medical image corresponding to the image sequence. According to an embodiment, an indication summary may be generated from the prior report using any suitable text processing approach. In a preferred embodiment of the invention, the data processing apparatus comprises a text mining module for this purpose. The indication summary can be regarded as a list of any abnormalities or findings previously noted for the prior study.

In order to identify a suitable image pair, an image pair selector may be applied. The image pair selector preferably deploys an image sorter configured to sort the images of a medical study or a medical image sequence into appropriate subsets. The types of subsets may depend on the medical imaging modality. For example, MRI images can be sorted into a pre-contrast sequence, a post-contrast sequence, etc. This may be done for medical imaging sequences of the prior study and of the current study. In particular, the images of the current study are sorted in the same manner as the images of the prior study.

The image pair selector may choose a medical image sequence, and a corresponding image, from the prior study on the basis of the indication summary and/or the determined findings. Each indication or finding noted in the prior report may be associated with a medical image in the prior image sequence. In particular, the image pair selector may choose the image sequence and/or the corresponding image from the prior study which most clearly shows the indication. This image is referred to herein as the "prior image" or "first image".

The image pair selector may choose the image from the current study that most clearly shows the determined finding and/or most closely matches the selected prior image, e.g. the image from the current study that corresponds to the same "slice" through the patient's body or the image from the current study which has been acquired by means of a similar image sequence. This image is referred to herein as the "current image" or "second image". Together, the first and second images constitute an image pair. The image pair selector can identify several image pairs, for example an image pair for each indication and/or finding noted in the prior report.

Even if the same MRI apparatus is used to generate the current and prior images, there may be various geometrical differences between the images of a pair, resulting for example from slight differences in how the patient lies on the scanner table, etc. Therefore, in a particularly preferred embodiment of the invention, the data processing apparatus preferably comprises a registration module configured to perform image registration on the images of an image pair. Image registration ensures that every pixel or voxel in the current image shows the same point in the patient's body as the corresponding pixel or voxel in the prior image. If that "slice" or 3D volume of the patient's body has not undergone any morphological change since the last medical imaging scan, the images of a pair should be essentially identical after image registration and intensity transformation.

The image pair may be input to the trained machine learning algorithm (MLA), which generates the change map. In the context of the invention, expressions such as "machine learning algorithm", "model", "encoder-decoder" and "encoder-decoder architecture" shall be understood to be synonyms and may be used interchangeably. Any contrastive-learning algorithm can be used. The machine learning algorithm may be assumed to comprise a neural network such as a deep learning neural network (DLNN). Any suitable DLNN architecture may be used, for example any suitable vision-based encoder-decoder.

As the skilled person will be aware, any publicly-available software for training a computer vision model which applies self-supervised learning (i.e. which does not require labelled training data or any metadata) can be used to train the MLA of the inventive longitudinal study comparator.

Preferably, the MLA learns features with a discriminative self-supervised method that combines two different losses, for example image-level losses and patch-level losses.

An example of such a product is DINOv2 (made available by Meta Research). Such freely-available methods can be applied at low cost to train a model to generate features that can then be used as inputs for a subsequent stage.

The accuracy of a machine learning algorithm depends to a large extent on the number of datasets with which it is trained. In a preferred embodiment of the invention, the machine learning algorithm is trained using at least several hundred training data sets, more preferably at least several thousand training data sets. Images used to train the machine learning algorithm can be obtained from one or more clinics. Equally, the machine learning algorithm could also be trained with synthetically generated images.

Training the machine learning algorithm preferably comprises the steps of:
A) compiling a training data set comprising a pair of images, namely an image from a current study and an equivalent image from an older study (same patient, same imaging modality, same task);
B) designing objectives for a contrastive-learning algorithm;
C) applying the contrastive-learning algorithm to the training data set to identify any differences between the images of the image pair; and
repeating steps A - C for a plurality of training data sets until a desired level of accuracy has been achieved, for example, until there an acceptably small variation between converged values of objectives (loss function) has been achieved.

A self-supervised training procedure of such a contrastive-learning algorithm preferably comprises a technique of backward-propagation to find the best set of parameters that will enable the network to infer a difference between the images, indicative of a development (an improvement or a deterioration) of the patient's condition between the prior study and the current study. The training procedure is preferably configured to achieve a favourably low cost function.

The inventive data processing apparatus, deploying an instance of the trained MLA, can then be used by a clinician to assist in quickly identifying any new development in the condition of the patient, for example an improvement such as a reduction in lesion size, or a deterioration such as increase in lesion size, appearance of a new lesion, etc. Even a very slight alteration vis-à-vis an older study can be quickly and accurately identified and presented as a change map, thereby significantly reducing the time required to accurately assess the current study. Instead of requiring an hour or more to perform a thorough comparison of images of a longitudinal study, the inventive approach allows a clinician to complete this task in only a few minutes.

Preferably, the change map that is presented to the clinician comprises a visual representation of any difference between the two studies and may be shown on a computer monitor. For example, each difference may be shown as a highlighted area overlaid on the corresponding image of the current study. The change map can be accompanied by a description giving the context for any noted difference. For example, the context for a highlighted area in the change map may be linked to the text of the corresponding indication or finding noted in the report of the prior study, and may also be linked to the corresponding prior image so that the clinician can quickly assess the extent of any development - an improvement or a deterioration - between the prior study and the current study. The clinician can then prepare a report for the current study by noting the identified alteration and augmenting this information by a diagnosis, an outlook, a treatment plan, etc. Such a report can also, at least partially, be prepared automatically by the inventive data processing apparatus.

The inventive longitudinal study comparator can be realized as a computer program to run on a processing unit connected to an imaging modality in a clinic. Equally, such a computer program can run on a remote server or cloud computing arrangement.

The following aspects are also part of the disclosure:
The invention also relates to a computer-implemented method for use in comparing medical images of a longitudinal study, which method comprises
- identifying an image pair, wherein an image pair comprises a first image from a medical image sequence of a prior study and a second image from a medical image sequence of a current study, and wherein the first image is selected on the basis of an indication noted in the prior study, and the second image corresponds sequentially to the first image;
- inputting the image pair to a machine learning algorithm previously trained to generate a change map indicating any difference between the current image and the prior image of the image pair; and
- outputting the change map to a user.

Preferably, the method comprises a step of performing text mining on a report of the prior study to obtain a summary of indications.

Preferably, a medical image sequence is obtained by magnetic resonance imaging.

Preferably, the machine learning algorithm is trained to generate a context for any indicated difference in the change map.

Preferably, the method comprises the steps of
- obtaining a current study comprising a medical image sequence;
   - obtaining a prior study comprising a medical image sequence and a report of noted indications;
- processing the medical image sequences using the computer-implemented method according to any of the previously described embodiments to obtain the change map.

Preferably, the method comprises a step of compiling a report for the current study on the basis of the change map.

The invention relates to a data processing apparatus adapted to perform the steps of the method according to any of the preceding embodiments, which data processing apparatus comprises
- an input interface for receiving the medical image sequence of the current study;
- a means of retrieving the prior study;
- a text processing module configured to generate an indication summary from the report of the prior study;
- an image pair selector configured to select an image pair comprising a first image from the prior study and a second image from the current study, by selecting the first image on the basis of the indication summary and by selecting the current image that corresponds to that prior image;
- a machine learning algorithm previously trained to generate a change map indicating any difference between the images of an image pair; and
- a means of outputting the change map to a user.

The invention relates to a data processing apparatus according to the preceding embodiment, comprising an image sorter configured to sort the images of a medical image sequence into image subsets on the basis of the medical imaging modality.

Preferably, the data processing apparatus is configured to perform image registration on the images of an image pair.

Preferably, the machine learning algorithm is realized as an image encoder-decoder.

The invention relates to a method of training the machine learning algorithm of the data processing apparatus according to any of the preceding embodiments, which method comprises at least the steps of
- generating an image pair from a medical image, wherein an image pair comprises modified versions of the medical image; and
- inputting the image pair to a machine-learning algorithm;
- applying self-supervised learning to the machine-learning algorithm to identify differences between the image pair.

Preferably, the step of generating an image pair comprises
- cropping the medical image to obtain a cropped image; and
- manipulating the intensity of the cropped image to obtain an intensity-manipulated cropped image.

Preferably, the method comprises a step of deploying an auxiliary model to calculate an image-level objective from the output of the machine-learning algorithm.

Preferably, the step of generating an image pair comprises
- cropping the medical image to obtain a cropped image; and
- masking a portion of the cropped image to obtain a masked image patch.

Preferably, the method comprises a step of deploying an auxiliary model to calculate a masked-image objective from the output of the machine-learning algorithm.

The embodiments and features described with reference to the method of the present invention apply mutatis mutandis to the device and/or the system of the present invention.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
- Figure 1: is a block diagram to illustrate a longitudinal study comparator;
- Figure 2: illustrates training of the MLA of Figure 1;
- Figures 3 - 5: are block diagrams of possible encoder-decoder architectures for the MLA trained as shown in Figure 2;
- Figure 6: is a flowchart to illustrate the invention.

In the diagrams, like numbers refer to like objects throughout. Objects in the diagrams are not necessarily drawn to scale. To improve the understandability of the present specification some of the described examples are only explained in the context of MRI imaging. However, the teachings can be likewise applied to other fields and technologies of medical imaging.

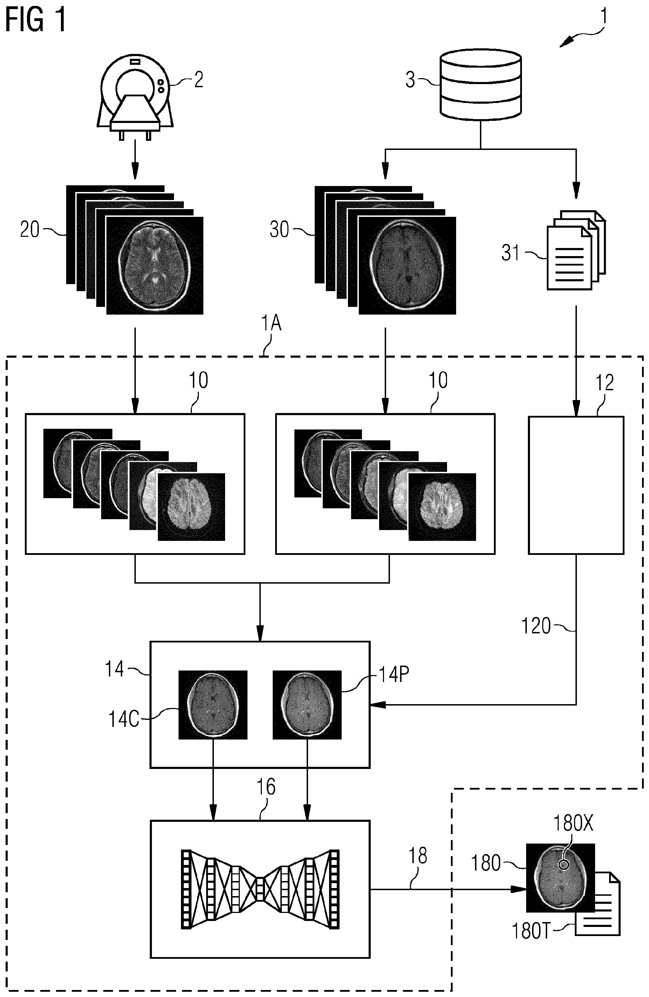

Figure 1 is a block diagram to illustrate the inventive approach to the comparison of medical images of a longitudinal study. The diagram shows a medical imaging apparatus 2 and image data 20 generated for a patient, e.g., a follow-up scan comprising multiple MRI images 20, referred to herein as the current image set or current study. Here, the medical imaging apparatus is an MRI scanner 2, and the follow-up scan is an MRI brain scan 20.

The diagram also shows a database 3 in which medical records are stored for that patient. A prior study 30 can be retrieved automatically or by a clinician, for example using the patient's identification number. Here, the prior study 30 comprises a similar image set 30, e.g., generated using the same type of imaging device but not necessarily the same device 2. The image set 30 of the prior study can therefore be assumed to comprise similar types of image sequences or series, for example a pre-contrast image series or sequence, a post-contrast image series or sequence, a gradient series or sequence, etc. The prior study also comprises a report 31, wherein the report in particular comprises a plurality of medical findings, in particular textual medical findings.

The longitudinal study comparator 1 comprises an image sorter 10 which identifies the various sequences or series present in an image set 20, 30 and sorts the images into those sequences or series, for example into pre-contrast images and post-contrast images. The diagram shows two instances of such a sorter 10 so that the image sets 20, 30 are sorted in parallel. Equally, a single instance of the sorter 10 can be used, so that the image sets 20, 30 are sorted sequentially.

In a preferably parallel path, the prior report 31 is analyzed by a text-mining algorithm 12, which can be realized as a language model, in particular a large language model, to identify any abnormalities or findings noted in the report at the time the prior study 30, 31 was made. The text-mining algorithm 12 outputs medical findings, preferably a list 120 or summary 120 of all abnormalities or findings identified in the report 31.

In a subsequent stage, a selector 14 chooses an image pair. The image pair comprises a first image 14P chosen from the prior image set 30 with the aid of the abnormality summary 120, i.e. the chosen image 14P will show an abnormality noted in the report 31 of the prior study.

A second image 14C of image pair is chosen from the current image set 20, and may be the image that most closely relates to the first image 14P and/or may be selected based on the at least one determined medical finding as well. For example, for 2D images, the second image 14C may show essentially the same "slice" of the patient's body. For 3D images, the first and second image may result of image sequences with similar characteristics. The selector 14 may perform image registration as necessary to facilitate a valid pixel-by-pixel (or voxel-by-voxel) comparison of the images 14C, 14P. Image registration ensures that each voxel of the current image 14C of the pair shows essentially the same point (in the patient's body) as the corresponding voxel of the prior image 14P.

Multiple image pairs 14C, 14P can be generated, depending on the number of indications or abnormalities identified by the text-mining algorithm 12. Each image pair 14C, 14P prepared in this way is then fed to a machine learning algorithm 16, previously trained to generate a change map 180 indicating any difference 180X between the current image 14C and the prior image 14P of a pair.

The change map 180 is presented to a user, for example in the form of an image 180 displayed on a monitor. The image 180 can show any difference 180X between the prior image 14P and the current image 14C of a pair, highlighted in any suitable manner. The change map 180 can be part of a change report 18 which also informs the user of the context, for example by including a text summary 180T referring to the abnormality previously noted in the prior report 31.

The machine learning algorithm may be an encoder-decoder 16 which maps the images 14C, 14P into a feature space to obtain a high-level description of the input data, and then generates the change map 180. The encoder-decoder 16 is preferably based on a deep learning neural network (DLNN) trained as explained above.

The study comparator 1 can significantly reduce the time required to assess the current MRI scan 20, since any development - whether a deterioration or an improvement - can be identified precisely and rapidly. Particularly in the case of a small change, for example a slight decrease in size of a lesion following successful treatment, or a slight increase in size of a tumor, the inventive method of comparing images of a longitudinal study can save much time, reducing the workload of radiologists and other clinicians.

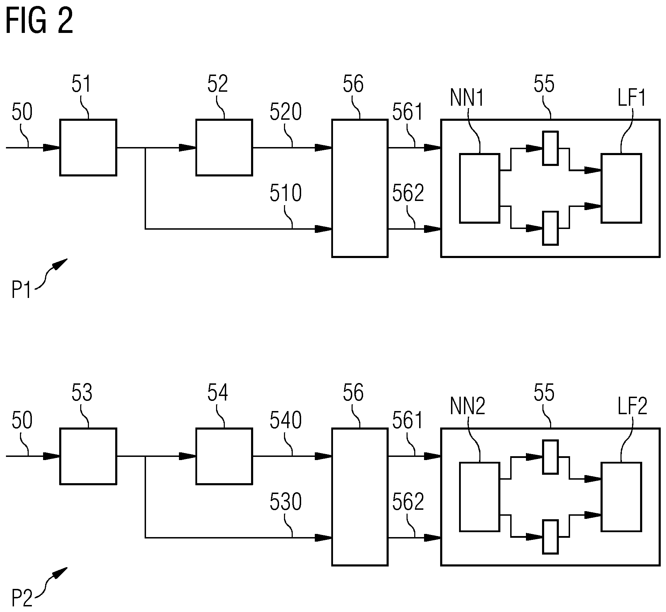

Figure 2 illustrates a preferred approach to training a machine learning algorithm 16 for the study comparator 1. Initially, an untrained model 56 is obtained. The untrained model 56 is an image encoder 56 comprising a student network and a teacher network. To train the model 56, any suitable medical images 50 can be used, for example MRI volumetric images. The inventive approach applies an image-level modelling path P1 and a masked-image modelling path P2 to train the MLA. Each path P1, P2 processes series of image pairs as explained in the following.

In the image-level modelling path P1, a single image 50 is cropped in block 51 to obtain a cropped image 510 (an image cropping step is not necessary in these paths P1, P2, but is a commonly performed step when training an image encoder). The cropped image 510 is transformed by intensity manipulation in block 52 to obtain a manipulated cropped image 520 (this stage can also include a step of spatial transformation with translation and/or rotation and/or flipping of the cropped image). The step of intensity manipulation can comprise altering the mean and/or standard deviation of the image intensity, for example. In this path P1, the manipulated cropped image 520 and the original cropped image 510 constitute an image pair.

In the masked-image modelling path P2, the image 50 is cropped in block 53 to obtain an image patch 530, which is then masked in block 54 to obtain a masked patch 540. A considerable percentage of the image patch 530 can be masked, for example up to 70%. In this path P2, the masked patch 540 and the original image patch 530 constitute an image pair.

Each image pair 510, 520; 530, 540 is used to train the model 56, for example using any publicly-available software for training a computer vision model using a self-supervised learning technique, as explained above. An image pair 510, 520; 530, 540 is processed by the image-encoder 56 and the processed patches 561, 562 are then used as input to a multi-layer perceptron 5 (also referred to as an "objective head") to calculate the respective objective. The objective heads 55 are used during the training. Each objective head 55 comprises an auxiliary model NN1, NN2 configured to process features extracted from the image encoder output 561, 562. The processed features are forwarded to an objective function or loss function LF1, LF2.

The output of each loss function LF1, LF2 is a number, i.e. the loss. The auxiliary model NN1 corresponds to the image-level objective of the image-level modelling path P1, while the auxiliary model NN2 corresponds to the masked-image objective of the masked-image modelling path P2 respectively. With this self-supervised training approach, which does not require any labelled input data, the initially untrained model 56 learns to extract similar features from each input image pair.

As the skilled person will be aware, to reduce the computing effort at the training or inference stage, parameters learnt by a larger model ("teacher") can be instilled into a smaller model ("student") in an approach referred to as knowledge distillation. In this exemplary embodiment, the model 56 is configured to comprise a teacher model and a (smaller) student model. Each of these models is a neural network.

In the image-level modelling path P1, the model 56 learns to disregard any intensity variations between the original cropped image 510 and the intensity-manipulated image 520. In this path, image-level losses are computed between features extracted from the student model and features extracted from the teacher model of the image encoder 56, whereby features from the student model are obtained by processing the manipulated image 520. The features from the teacher model are obtained by processing the cropped image 510. In this way, the model 56 learns (i.e. is trained) to extract similar features independent of the intensity distribution of the input images 510, 520.

In the masked-image modelling path P2, the student model of the MLA 56 receives masked patches 540, and the teacher model receives the original patches 530. In this path, mask-level losses are computed between features extracted from the student model and features extracted from the teacher model of the image encoder 56, whereby features from the student model are obtained by processing the manipulated image 540. The features from the teacher model are obtained by processing the cropped image 530. In this way, the model 56 learns (i.e. is trained) to extract similar features from the input images 530, 540 even if part of the input data is missing.

Training of the model 56 can be done with many image pairs. After completion, the trained model is ready for use, for example as the MLA 16 of Figure 1.

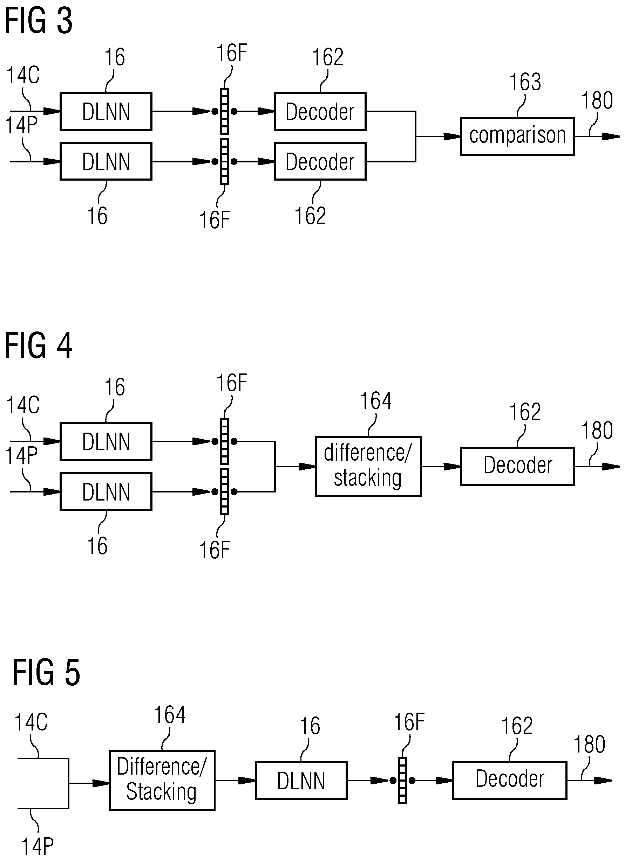

Figures 3 - 5 show various possible encoder-decoder architectures. Each version is configured to receive an image pair 14C, 14P and to output a change map 180. Each architecture comprises a deep learning neural network, DLNN, 16 trained as explained in Figure 2. The DLNN 16 in each case generates feature vectors 16F representing the input data, i.e. the model 16 has learned to map the input data to feature vectors 16F. Each architecture deploys a decoder 162 which maps feature vectors 16F to meaningful data.

In the architecture of Figure 3, each decoder 162 outputs a change map or a findings map showing the findings in each image. Both findings maps or change maps are compared in a comparator 163, which in turn outputs the final change map 180 (for that image pair 14C, 14P) to the user. As the findings maps are compared with each other, each findings map is human readable, such that insights into the generation process can be effectively generated.

In the architecture of Figure 4, the feature vectors 16F are subtracted or concatenated in a difference/stacking stage 164 and then passed to the decoder 162, which in turn outputs the change map 180. As the change map is determined based on the features vectors 16F, this approach yields precise results, while being computationally effective.

The architecture of Figure 5 illustrates a different sequence of steps, with a difference/stacking stage 164 preceding the model 16. In this case also, the decoder 162 receives the feature vectors 16F and outputs the change map 180. To improve the results of the comparison, this approach is preferably combined with a registration step, such that the images to be compared have similar or the same dimensions.

In each case, a change map 180 will highlight any difference between the images 14C, 14P of an image pair. The images 14C, 14P of an image pair correspond to a similar image sequence, and different image pairs represent different medical sequences. In one embodiment, the change map may be calculated based on 3D data, and 2D images, also called "slices" may be derived from the image data, as explained above. If a slice has not undergone any morphological change since the last medical imaging scan, the corresponding slices of the images 14C, 14P should be identical, the summary accompanying the change map 180 can report "no change". If a morphological change has taken place since the last medical imaging scan, the relevant slices of the images 14C, 14P will differ, and the summary accompanying the change map 180 will report any changes between the images. As explained in Figure 1, the change map 180 can be shown to the clinician on a display or monitor, with any development annotated with a visual highlight 180X. The summary 180T accompanying the change map 180 can include helpful text, for example any relevant sections of the prior report 31.

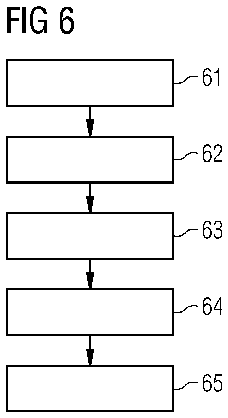

Figure 6 shows an exemplary flowchart to illustrate the inventive approach to performing a longitudinal study comparison, using the inventive data processing apparatus. In a first step 61, the medical image sequence (for example an MRI sequence) of a current study is obtained. In a second step 62, an equivalent medical image sequence and report of a previous study are obtained. In a subsequent step 63, both studies are fed to an MLA previously trained as explained above to identify any differences between the prior study and the current study. The MLA generates a change map from which, in step 64, the clinician can readily see any relevant differences between the older study and the current study. For example, a difference between volume data of the MRI scans can have been identified on the basis of an indication noted in the report of the prior study, and the relevant region can be shown to the clinician by highlighting it in an image of the current study. In a final step 65, the clinician may instruct the data processing apparatus to generate a report for the current study in which any such differences are noted.

For the sake of clarity, it is to be understood that the use of "a" or "an" throughout this application does not exclude a plurality, and "comprising" does not exclude other steps or elements. The mention of a "unit" or a "module" does not preclude the use of more than one unit or module. Any pronoun denoting a specific gender shall be understood to apply equally to any gender identity.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims. Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc., may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the spirit or scope of the subject matter disclosed herein. Thus, the present disclosure is not intended to be limited to the embodiments shown herein but is to be accorded the widest scope consistent with the following claims and the principles and novel features disclosed herein. While various aspects and embodiments have been disclosed, other aspects and embodiments are contemplated. The various aspects and embodiments disclosed are for purposes of illustration and are not intended to be limiting, with the true scope and spirit being indicated by the following claims.

## Claims

1. Computer-implemented method for generating a change map indicating at least one difference between a current image and a prior image, which method comprises
- obtaining a plurality of prior medical image sequences (30) from a prior study (30, 31), and a prior report (31) from the prior study (30,31);
- obtaining a plurality of current medical image sequences (20) from a current study;
- determining at least one medical finding based on the prior report;
- identifying, based on the determined medical finding, an image pair (14P, 14C), wherein the image pair (14P, 14C) comprises a prior image (14P) from one of the prior medical image sequences (30) and a current image (14C) from one of the current medical image sequences (20), and
- inputting the image pair (14P, 14C), and optionally the prior report (31), to a machine learning algorithm (16) trained to generate a change map (180) indicating at least one difference (180X) between the current image (14C) and the prior image (14P) of the image pair (14P, 14C).

2. Method according to the preceding claim, wherein the method comprises a step of outputting the change map (180) to a user and/or providing the change map (180) for further processing.

3. Method according to any one of the preceding claims, wherein the method comprises a step of performing text mining on the prior report (31) to determine the at least one medical finding.

4. Method according to any one of the preceding claims, wherein the prior image (14P) is determined based on the medical finding and the current image (14C) corresponds sequentially to the prior image.

5. Method according to any one of the preceding claims, wherein the method comprises a step of generating a context (180X) for a difference indicated in the change map based on the image pair (14P, 14C), the prior report (31) and/or based on the determined at least one medical finding, preferably by means of the machine learning algorithm, wherein, preferably, the machine learning algorithm (16) is trained to generate the context (180T) for at least one difference (180X) indicated in the change map (180).

6. A method according to the preceding claim, comprising a step of compiling a report (21) for the current study (20) on the basis of the change map (180).

7. Method according to any one of the preceding claims, wherein the prior image (14P) and/or the current image (14C) is a 3D medical image.

8. Method according to any one of the preceding claims, wherein the method comprises a step of
- Obtaining a raw image set of the prior study and/or a raw image set of the current study;
- Sorting the medical images of the raw image set into medical image sequences, based on the sequence used to acquire the medical images.

9. Method according to any one of the preceding claims, wherein the method comprises a step of conducting image registration on the prior image (14P) and/or on the current image (14C).

10. Method according to any one of the preceding claims, wherein the prior medical image (14P) and/or the current medical image (14C) is obtained by magnetic resonance imaging.

11. Method according to any one of the preceding claims, wherein the prior study and/or the current study was conducted under administration of a contrast agent, and the prior image (14P) and/or the current image (14C) is further identified based on the contrast setting of the corresponding image sequence.

12. Method according to any one of the preceding claims, wherein a summary of medical findings is generated based on the prior report, and wherein at least one of the following steps is repeated for each of the medical findings in the summary of medical findings:
- identifying, based on the determined medical finding, an image pair (14P, 14C), wherein the image pair (14P, 14C) comprises a prior image (14P) from one of the medical image sequences (30) of the prior study (30, 31) and a current image (14C) from one of the medical image sequences (20) of the current study,
- inputting the image pair (14P, 14C), and optionally the prior report (31), to a machine learning algorithm (16) trained to generate a change map (180) indicating at least one difference (180X) between the current image (14C) and the prior image (14P) of the image pair (14P, 14C); and
- outputting the change map (180) to a user or providing the change map (180) for further processing.

13. A data processing apparatus comprising means for carrying out the steps of the method of any of claims 1 to 12.

14. A computer program product comprising instructions which, when the program is executed by a computer, cause the computer to carry out the steps of the method of any of claims 1 to 12.

15. A computer-readable storage medium comprising instructions which, when executed by a computer, cause the computer to carry out the steps of the method of any of claims 1 to 12.
